# EUROPÄISCHE PATENTANMELDUNG

(11) **EP 3 178 828 A1**
(43) Veröffentlichungstag der Anmeldung: **14.06.2017**
(21) Anmeldenummer: 15198153.7
(22) Anmeldetag: 07.12.2015
(51) Int. Cl.: C07F 9/6574

(54) **HETEROZYKLISCHE SELENA-PHOSPHITE SOWIE VERFAHREN ZU DEREN HERSTELLUNG**

(71) Anmelder: Evonik Degussa GmbH, 45128 Essen (DE)
(72) Erfinder: DYBALLA, Katrin Marie, 45657 Recklinghausen (DE); FRANKE, Robert, 45772 Marl (DE); WEILBEER, Claudia, 06406 Bernburg (DE); SELENT, Detlef, 18059 Rostock (DE); BÖRNER, Armin, 18059 Rostock (DE)

(57) **Zusammenfassung**

Neue heterozyklische Selena-Phosphite, Verfahren zu deren Herstellung als auch deren Verwendung als Liganden für eine Anwendung in Komplexen.

## Beschreibung

Neue heterozyklische Selena-Phosphite, Verfahren zu deren Herstellung als auch deren Verwendung als Liganden für eine Anwendung in Komplexen.

T. K. Paine beschreibt eine Synthese von 2,2'-Selenobis(4,6-di-tert-butylphenol) unter Verwendung von Selendioxid. Die Herstellung von 2,2'-Selenobis(4,6-di-tert-butylphenol) erfolgt hier in einem sauren Medium unter Zusatz von konzentrierter Salzsäure. Das Produkt wird mit einer Ausbeute von 25 % erhalten (T. K. Paine et al., "Manganese complexes of mixed O, X, O-donor ligands (X = S or Se): synthesis, characterization and catalytic reactivity", Dalton Trans., 2003, 15, 3136-3144). Hierbei ist besonders nachteilig, dass die Ausbeuten sehr gering und somit verbesserungswürdig sind.

Eine weitere mehrstufige Syntheseroute unter Verwendung von Grignard-Reagenz offenbart H. M. Lin et al., "A novel and efficient synthesis of selenides", ARKIVOC, 2012, viii, 146-156. Es wird eine Syntheseroute für Selenobiarylether offenbart in der zunächst Brom an das entsprechende Phenol addiert werden muss, um das Produkt dann mit Magnesium zu einem Grignard-Reagenz umzusetzen. Das Grignard-Reagenz kann dann mit dem zugesetzten Selen reagieren, bevor die eigentliche Kupplung zum Biarylether erfolgt:

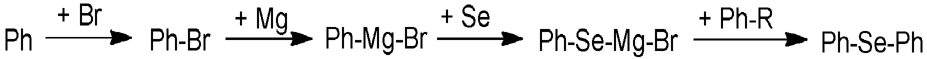

Das Produkt konnte in einer guten Ausbeute erzielt werden, jedoch ist diese Syntheseroute sehr aufwendig, was es für einen großtechnischen Einsatz unattraktiv macht. Hierbei sind eine Vielzahl von Syntheseschritten notwendig, die zum Teil nicht unkritisch in ihrer Durchführung sind, insbesondere wenn man an eine Hochskalierung denkt und Massstäbe verwendet, die in der Industrie üblich sind. Weiterhin fallen bei dieser Syntheseroute große Mengen an Abfallprodukten und Lösungsmittel an, die aufwändig entsorgt werden müssen, unter anderem auf Grund des Einsatzes von Brom.

Eine grosstechnisch wirtschaftliche Syntheseroute zur Herstellung von Selenodiphenolen beschreibt die EP 15168645.8 bzw. US 14/720,063.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxidierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021 /cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Rhodium-Monophosphit-Komplexe in katalytisch aktiven Zusammensetzungen sind geeignet für die Hydroformylierung von verzweigten Olefinen mit innenständigen Doppelbindungen. Seit den 1970er Jahren ist die Verwendung von so genannten "bulky phosphites" in der Hydroformylierung beschrieben (siehe u.a. van Leeuwen et al., Journal of Catalysis, 2013, 298, 198-205). Diese zeichnen sich durch eine gute Aktivität aus, jedoch ist die n/i-Selektivität für endständig oxidierte Verbindungen gering und verbesserungswürdig.

In diesen Hydroformylierungen werden in der Regel Mono- und Bisphosphite eingesetzt, die oftmals aus Biphenol-Bausteinen aufgebaut sind. Die Entwicklung neuer Liganden ist häufig durch die zur Verfügung stehenden Biphenol-, also Ligandenbausteine limitiert. So stellen 2,2'-Selenobiarylether sowie Diphenylselenoxide und Diphenylselenide eine hochinteressante Verbindungsklasse dar. Die 2,2'-Selenobiarylether werden derzeit nur in bestimmten Komplexen, vor allem manganhaltigen, verwendet, sie besitzen aber ein großes Potential für weitere Anwendungen.

Aufgabe der Erfindung war es, eine weitere gänzlich neue Substanzklasse an Liganden und Ligandenbausteinen herzustellen, um das Feld der verfügbaren Liganden für die jeweiligen spezifischen Komplexe in der Katalyse zu erweitern. Des Weiteren bestand die Aufgabe Liganden für Rhodium-Hydroformylierungskatalysatoren herzustellen. Daher bestand auch die Aufgabe neue Zwischenprodukte als Ligandenbausteine zur Herstellung von Liganden bereitzustellen. Gelöst werden die Aufgaben mit den heterozyklischen Selena-Phosphiten nach Anspruch 1, dem Verfahren nach Anspruch 6 sowie der Verwendung nach Anspruch 14. Besondere Ausführungsformen sind in den Unteransprüchen sowie detailliert in der Beschreibung offenbart. Gelöst werden die Aufgaben vorzugsweise mit Selena-Phosphiten der Strukturen **I** und **Ia**, insbesondere mit R¹ ausgewählt aus Struktur **II**, **VII**, **VIII**, **IX** und **X**. Dabei sind die Wasserstoff, Alkyl und -O-(C₁-C₁₂)-Alkyl substituierten Verbindungen von R¹ der genannten Strukturen besonders bevorzugte Verbindungen.

Gegenstand der Erfindung sind Verbindungen eines heterozyklischen Selena-Phosphites mit einer allgemeinen Struktur **I** 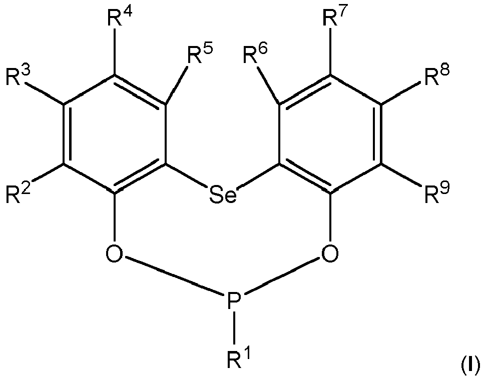 wobei R², R³, R⁴, R⁵, R⁶, R⁷, R⁸ und R⁹ jeweils unabhängig voneinander ausgewählt sind aus: -H,-(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₂H, -CN, -N[(C₁-C₁₂)-Alkyl]₂, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe mindestens einen Substituenten aufweisen kann und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei -R¹ unabhängig ausgewählt ist aus -O-(C₆-C₂₀)-Aryl, -O-(C₆-C₂₀)-Aryl-(C₁-C₁₂)-Alkyl, -O-(C₆-C₂₀)-Aryl-O-(C₁-C₁₂)-Alkyl, -O-(C₆-C₂₀)-Aryl-(C₆-C₂₀)-Aryl, -O-(C₆-C₂₀)-Aryl-O-(C₆-C₂₀)-Aryl, -O-(C₁-C₁₂)-Alkyl, -O-(C₁-C₁₂)-Alkyl-O-(C₁-C₁₂)-Alkyl, -O-(C₁-C₁₂)-Alkyl-(C₆-C₂₀)-Aryl, -O-(C₃-C₁₂)-Cycloalkyl, wobei Alkyl jeweils unabhängig linear, cyclisch oder verzweigt ist, wobei die genannten Alkyl- und Arylgruppen jeweils unabhängig unsubstituiert oder substituiert sind, die jeweilige substituierte-(C₆-C₂₀)-Arylgruppe weist mindestens einen oder mehrere Substituenten auf; wobei die Substituenten an der jeweiligen Arylgruppe unabhängig voneinander ausgewählt sein kann aus: -O-(C₁-C₁₂)-Alkyl, -(C₁-C₁₂)-Alkyl-O-(C₁-C₁₂)-Alkyl, -(C₁-C₁₂)-Alkyl, -O-(C₆-C₂₀)-Aryl, -O-(C₁-C₁₂)-Alkyl-O-(C₁-C₁₂)-Alkyl, -O-(C₆-C₂₀)-Aryl-O-(C₁-C₁₂)-Alkyl, -(C₃-C₁₂)-Cycloalkyl, -(C₃-C₁₂)-Heterocycloalkyl, -(C₆-C₂₀)-Aryl, Cyano, -Halogen, -O(C=O)-O-(C₁-C₁₂)-Alkyl, -N[(C₁-C₁₂)-Alkyl]₂.

Nach besonders bevorzugten Alternativen kann R¹ in der Struktur **I** ausgewählt sein aus Verbindungen der nachfolgenden Strukturen **II**, **III**, **IV**, **V**, **VI**, **VII**, **VIII**, **IX** und **X**. R¹ in der ist ein -O-verbrückter organofunktioneller Rest. Dabei ist **IX** ein Cyclododecanyl- und **X** ein Menthyl-Rest. 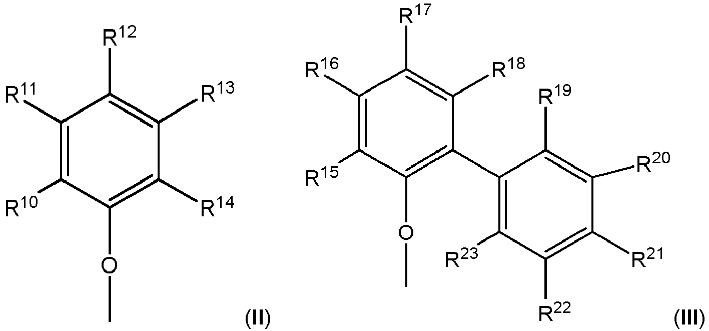 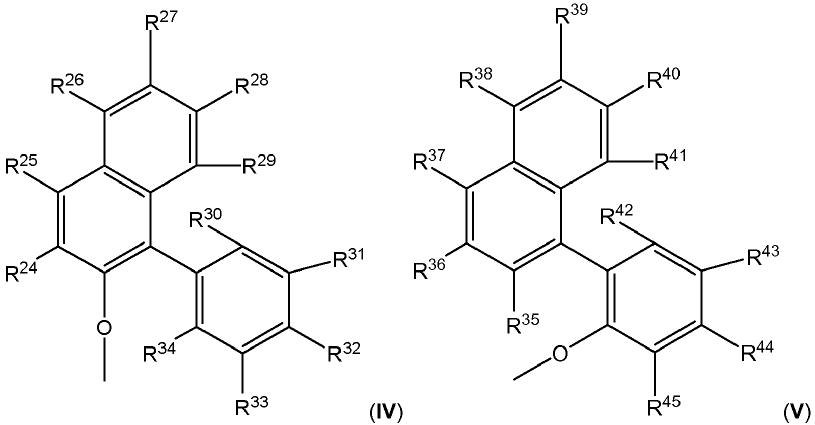 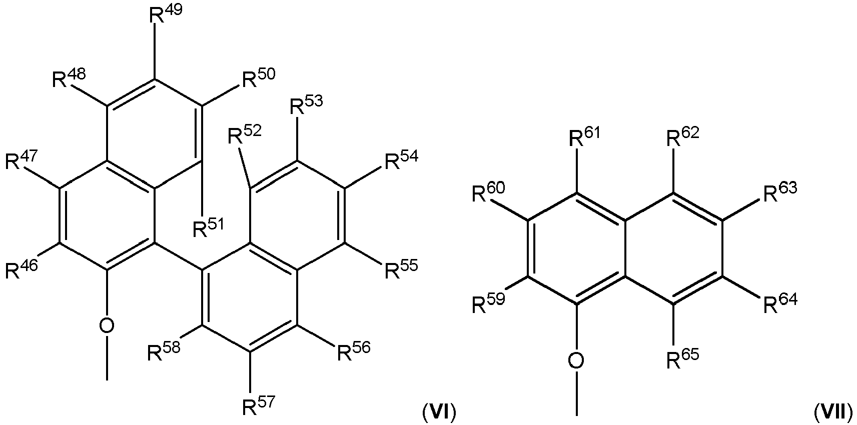 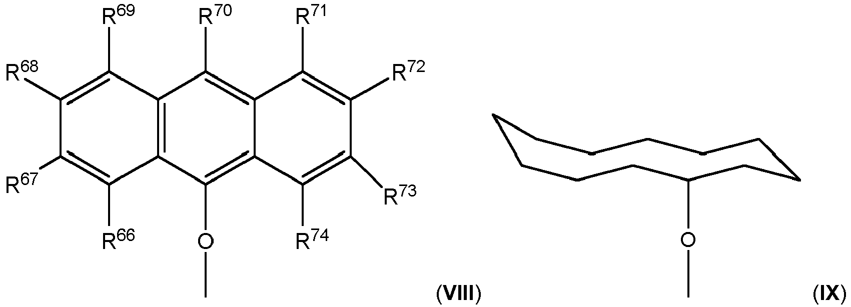 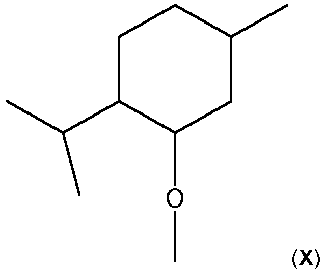 wobei die Reste R¹⁰, R¹¹, R¹², R¹³, R¹⁴ in der Struktur **II**, R¹⁵, R¹⁶, R¹⁷, R¹⁸, R¹⁹, R²⁰, R²¹, R²² und R²³ in Struktur **III**, R²⁴, R²⁵, R²⁶, R²⁷, R²⁸, R²⁹, R³⁰, R³¹, R³², R³³ und R³⁴ in Struktur **IV**, R³⁵, R³⁶, R³⁷, R³⁸, R³⁹, R⁴⁰, R⁴¹, R⁴², R⁴³, R⁴⁴ und R⁴⁵ in Struktur **V**, R⁴⁶, R⁴⁷, R⁴⁸, R⁴⁹, R⁵⁰, R⁵¹, R⁵², R⁵³, R⁵⁴, R⁵⁵, R⁵⁶, R⁵⁷ und R⁵⁸ in Struktur **VI**, R⁵⁹, R⁶⁰, R⁶¹, R⁶², R⁶³, R⁶⁴ und R⁶⁵ in Struktur **VII**, sowie R⁶⁶, R⁶⁷, R⁶⁸, R⁶⁹, R⁷⁰, R⁷¹, R⁷², R⁷³ und R⁷⁴ in Struktur **VIII**,
in jeder Struktur jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkyl- und Arylgruppen jeweils unabhängig unsubstituiert oder substituiert sein können, wobei die jeweilige substituierte -(C₁-C₁₂)-Alkylgruppe und die jeweilige substituierte -(C₆-C₂₀)-Arylgruppe mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
wobei in den Strukturen **III, IV, V** und **VI** R²³, R³⁴, R⁴⁵, R⁵⁸ jeweils unabhängig zusätzlich ausgewählt sein können aus -O-X mit X gleich Schutzgruppe, wobei die Schutzgruppe X ausgewählt sein kann aus -(C₁-C₁₂)-Alkyl, -(C₁-C₁₂)-Alkyl-O-(C₁-C₁₂)-Alkyl, -(C₆-C₂₀)-Aryl-O-(C₁-C₁₂)-Alkyl, -COO-(C₁-C₁₂)-Alkyl.

Nach einer weiteren besonders bevorzugten Alternative kann das heterozyklische Selena-Phosphit die allgemeine Struktur **Ia** aufweisen 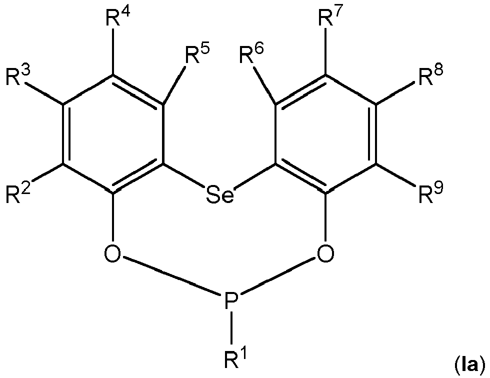 wobei R², R³, R⁴, R⁵, R⁶, R⁷, R⁸ und R⁹ jeweils in der Struktur **Ia** unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl,-Halogen, und
mit -R¹ im heterozyklischen Selena-Phosphit der allgemeinen Struktur **Ia** unabhängig ausgewählt ist aus den Strukturen **II**, **III**, **IV**, **V**, **VI**, **VII**, **VIII**, **IX** und **X**, wie vorstehend dargestellt, wobei die Reste
R¹⁰, R¹¹, R¹², R¹³, R¹⁴ in der Struktur **II**, R¹⁵, R¹⁶, R¹⁷, R¹⁸, R¹⁹, R²⁰, R²¹ und R²² in Struktur **III**, R²⁴, R²⁵, R²⁶, R²⁷, R²⁸, R²⁹, R³⁰, R³¹, R³² und R³³ in Struktur **IV**, R³⁵, R³⁶, R³⁷, R³⁸ R³⁹ R⁴⁰, R⁴¹, R⁴², R⁴³ und R⁴⁴ in Struktur **V**, und/oder R⁴⁶, R⁴⁷, R⁴⁸, R⁴⁹, R⁵⁰, R⁵¹, R⁵², R⁵³, R⁵⁴, R⁵⁵, R⁵⁶, und R⁵⁷ in Struktur **VI,** R⁵⁹, R⁶⁰, R⁶¹, R⁶², R⁶³, R⁶⁴ und R⁶⁵ in Struktur **VII,** sowie R⁶⁶, R⁶⁷, R⁶⁸, R⁶⁹, R⁷⁰, R⁷¹, R⁷², R⁷³ und R⁷⁴ in Struktur **VIII,** jeweils unabhängig voneinander ausgewählt sein können aus: -H, -(C₁-C₁₂)-Alkyl, -O-(C₁-C₁₂)-Alkyl, -Halogen, und wobei jeweils unabhängig zusätzlich zu den vorgenannten Gruppen in den Strukturen **III, IV, V** und **VI** R²³, R³⁴, R⁴⁵, R⁵⁸ jeweils unabhängig ausgewählt sein können aus -H,
-(C₁-C₁₂)-Alkyl, -Halogen und -O-X mit X gleich Schutzgruppe, wobei die Schutzgruppe X ausgewählt ist aus -(C₁-C₁₂)-Alkyl, -(C₁-C₁₂)-Alkyl-O-(C₁-C₁₂)-Alkyl, -(C₆-C₂₀)-Aryl-O-(C₁-C₁₂)-Alkyl, -COO-(C₁-C₁₂)-Alkyl.

Entsprechend einer weiteren bevorzugten Alternative kann das heterozyklische Selena-Phosphit der allgemeinen Struktur **Ia** ausgewählt sein aus mindestens einer Verbindung der Struktur **Ib** mit R¹ entsprechend der Definition für die Verbindung der Struktur **Ia** 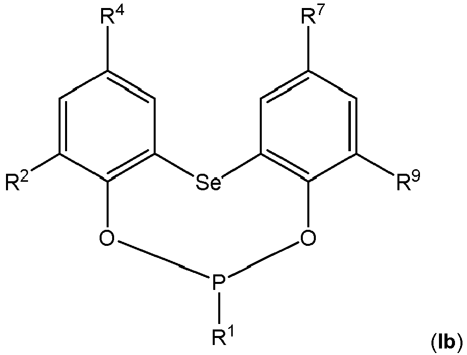 wobei R², R⁴, R⁷ und R⁹ jeweils unabhängig voneinander ausgewählt sein können aus: -(C₁-C₁₂)-Alkyl, -O-(C₁-C₁₂)-Alkyl, -(C₆-C₂₀)-Aryl, -O-(C₆-C₂₀)-Aryl, -Halogen.

Gleichfalls Gegenstand der Erfindung sind in den vorgenannten Strukturen der Selena-Phosphite und Selenodiarylen der Strukturen **I** und **Ia** mit R², R³, R⁴, R⁵, R⁶, R⁷, R⁸ und R⁹, die jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkyl- und Arylgruppen jeweils unabhängig unsubstituiert sind. Vorzugsweise sind die Alkylgruppen unsubstituiert. Besonders bevorzugt sind R², R³, R⁴, R⁵, R⁶, R⁷, R⁸ und R⁹ jeweils unabhängig voneinander ausgewählt aus Methyl-, Ethyl-, *tert*-Butyl-, iso-Pentyl, Methoxy-.

Ferner ist Gegenstand der Erfindung sind in den vorgenannten Strukturen der Selena-Phosphite und Selenodiarylen der Strukturen **Ib** mit R², R⁴, R⁷ und R⁹, die jeweils unabhängig voneinander ausgewählt sein können aus: -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkyl- und Arylgruppen jeweils unabhängig unsubstituiert sind.

In einer Alternative sind R², R³, R⁴, R⁵, R⁶, R⁷, R⁸ und R⁹ in einem heterozyklischen Selena-Phosphit der allgemeinen Struktur **I**, und **Ia** jeweils unabhängig voneinander ausgewählt aus: -H und -(C₁-C₁₂)-Alkyl und/oder -O-(C₁-C₁₂)-Alkylgruppen, wobei die Alkylgruppen linear, verzweigt oder cyclisch sein können. Vorzugsweise sind die Alkylgruppen unsubstituiert.

In einer Alternative sind R², R⁴, R⁷ und R⁹ in einem heterozyklischen Selena-Phosphit der allgemeinen Struktur **Ib** jeweils unabhängig voneinander ausgewählt aus:
-(C₁-C₁₂)-Alkyl und/oder -O-(C₁-C₁₂)-Alkylgruppen, wobei die Alkylgruppen linear, verzweigt oder cyclisch sein können. Vorzugsweise sind die Alkylgruppen unsubstituiert. Besonders bevorzugt sind R², R⁴, R⁷, R⁹ die jeweils Methyl-, Ethyl-, *tert*-Butyl-, iso-Pentyl, Methoxy- sein können.

Dabei ist es für die heterozyklischen Selena-Phosphite der vorgenannten allgemeinen Struktur **Ia** oder **Ib** weiter bevorzugt, wenn R¹ einer der Strukturen ausgewählt aus **II**, **III**, **IV**, **V**, **VI**, **VIII**, **IX** und **X** entsprechen kann, wobei die Strukturen **II**, **III,** besonders bevorzugt sind und die Reste in den Strukturen ausgewählt sind aus:
R¹⁰, R¹¹, R¹², R¹³, R¹⁴ in der Struktur **II**,
R¹⁵, R¹⁶, R¹⁷, R¹⁸, R¹⁹, R²⁰, R²¹, R²² und R²³ in Struktur **III**,
R²⁴, R²⁵, R²⁶, R²⁷, R²⁸, R²⁹, R³⁰, R³¹, R³², R³³ und R³⁴ in Struktur **IV**,
R³⁵, R³⁶, R³⁷, R³⁸, R³⁹, R⁴⁰, R⁴¹, R⁴², R⁴³, R⁴⁴ und R⁴⁵ in Struktur **V**, und/oder
R⁴⁶, R⁴⁷, R⁴⁸, R⁴⁹, R⁵⁰, R⁵¹, R⁵², R⁵³, R⁵⁴, R⁵⁵, R⁵⁶, R⁵⁷ und R⁵⁸ in Struktur **VI**,
R⁵⁹, R⁶⁰, R⁶¹, R⁶², R⁶³, R⁶⁴ und R⁶⁵ In Struktur **VII**, sowie
R⁶⁶, R⁶⁷, R⁶⁸, R⁶⁹, R⁷⁰, R⁷¹, R⁷², R⁷³ und R⁷⁴ in Struktur **VIII**,
in jeder Struktur jeweils unabhängig voneinander ausgewählt sein können aus: -H, -(C₁-C₁₂)-Alkyl, -Halogen, wobei die Alkylgruppen jeweils unabhängig unsubstituiert sind, wobei in den Strukturen **III**, **IV**, **V** und **VI** R²³, R³⁴, R⁴⁵, R⁵⁸ jeweils unabhängig ausgewählt sein können aus -H, -(C₁-C₁₂)-Alkyl, -O-X mit X gleich Schutzgruppe, wobei die Schutzgruppe X ausgewählt sein kann aus -(C₁-C₁₂)-Alkyl, -(C₆-C₂₀)-Aryl-O-(C₁-C₁₂)-Alkyl, -COO-(C₁-C₁₂)-Alkyl.

In Struktur **II** sind R¹⁰ und R¹² vorzugsweise ausgewählt aus *tert*-Butyl, Methyl-, Ethyl-, iso-Propy, iso-Pentyl- und R¹¹, R¹³, R¹⁴ sind -H.

Ebenfalls beansprucht wird mindestens ein heterozyklisches Selena-Phosphit der allgemeinen Struktur **I**, wobei R², R³, R⁴, R⁵, R⁶, R⁷, R⁸ und R⁹ jeweils unabhängig voneinander ausgewählt sind aus: -H, -(C₁-C₁₂)-Alkyl, -O-(C₁-C₁₂)-Alkyl, und -R¹ im heterozyklischen Selena-Phosphit der allgemeinen Struktur **I** unabhängig ausgewählt ist aus den Strukturen **II**, **III**, **IV**, **V**, **VI**, **VII**, **VIII**, **IX** und **X**, wobei die vorgenannten Reste jeweils unabhängig voneinander ausgewählt sind aus: -H,-(C₁-C₁₂)-Alkyl, -O-(C₁-C₁₂)-Alkyl, und wobei jeweils unabhängig zusätzlich zu den vorgenannten Resten in den Strukturen **III, IV, V** und **VI** R²³, R³⁴, R⁴⁵, R⁵⁸ jeweils unabhängig ausgewählt sind aus -H, -(C₁-C₁₂)-Alkyl, und -O-X mit X gleich Schutzgruppe, wobei die Schutzgruppe X ausgewählt ist aus -(C₁-C₁₂)-Alkyl, -(C₆-C₂₀)-Aryl, -(C₁-C₁₂)-Alkyl-O-(C₁-C₁₂)-Alkyl, -(C₆-C₂₀)-Aryl-O-(C₁-C₁₂)-Alkyl.

Ebenfalls beansprucht wird mindestens ein heterozyklisches Selena-Phosphit der allgemeinen Struktur **I**, wobei R², R³, R⁴, R⁵, R⁶, R⁷, R⁸ und R⁹ jeweils unabhängig voneinander ausgewählt sind aus: -H, -(C₁-C₁₂)-Alkyl, -O-(C₁-C₁₂)-Alkyl, und -R¹ im heterozyklischen Selena-Phosphit der allgemeinen Struktur **I** unabhängig ausgewählt ist aus den Strukturen **II**, **VII**, **VIII**, wobei die Reste an **II**, **VII, VIII** jeweils unabhängig voneinander ausgewählt sind aus: -H, -(C₁-C₁₂)-Alkyl, -O-(C₁-C₁₂)-Alkyl.

Ebenfalls beansprucht wird mindestens ein heterozyklisches Selena-Phosphit der allgemeinen Struktur **I**, wobei R², R³, R⁴, R⁵, R⁶, R⁷, R⁸ und R⁹ jeweils unabhängig voneinander ausgewählt sind aus: -H, -(C₁-C₁₂)-Alkyl, , und -R¹ im heterozyklischen Selena-Phosphit der allgemeinen Struktur **I** unabhängig ausgewählt ist aus der Struktur **II**, wobei die Reste an **II** jeweils unabhängig voneinander ausgewählt sind aus: -H, -(C₁-C₁₂)-Alkyl.

Ebenfalls beansprucht wird mindestens ein heterozyklisches Selena-Phosphit der allgemeinen Struktur **I**, wobei R², R³, R⁴, R⁵, R⁶, R⁷, R⁸ und R⁹ jeweils unabhängig voneinander ausgewählt sind aus: -H, -(C₁-C₁₂)-Alkyl, -O-(C₁-C₁₂)-Alkyl, und -R¹ im heterozyklischen Selena-Phosphit der allgemeinen Struktur **I** unabhängig ausgewählt ist aus den Strukturen **IX** und **X.**

Besonders bevorzugt ist ein heterozyklisches Selena-Phosphit der allgemeinen Struktur **Ib**, wobei R², R⁴, R⁷ und R⁹ jeweils Methyl sind und -R¹ der Struktur **II** entspricht mit R¹⁰, R¹² gleich Methyl und R¹¹, R¹³ und R¹⁴ gleich -H.

Ferner ist Gegenstand der Erfindung ein Verfahren zur Herstellung mindestens eines heterozyklische Selena-Phosphits der allgemeinen Struktur **I** entsprechend vorstehender Definition sowie die Selena-Phosphite erhältlich nach dem Verfahren, 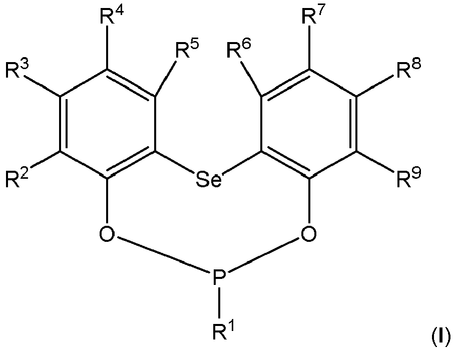 wobei R², R³, R⁴, R⁵, R⁶, R⁷, R⁸ und R⁹ jeweils unabhängig voneinander ausgewählt sind aus: -H,-(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₂H, -CN, -N[(C₁-C₁₂)-Alkyl]₂, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe, mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und wobei -R¹ unabhängig ausgewählt sein kann aus: -O-(C₆-C₂₀)-Aryl, -O-(C₆-C₂₀)-Aryl-(C₁-C₁₂)-Alkyl, -O-(C₆-C₂₀)-Aryl-O-(C₁-C₁₂)-Alkyl, -O-(C₆-C₂₀)-Aryl-(C₆-C₂₀)-Aryl, -O-(C₆-C₂₀)-Aryl-O-(C₆-C₂₀)-Aryl, -O-(C₁-C₁₂)-Alkyl, -O-(C₁-C₁₂)-Alkyl-O-(C₁-C₁₂)-Alkyl, -O-(C₁-C₁₂)-Alkyl-(C₆-C₂₀)-Aryl, -O-(C₃-C₁₂)-Cycloalkyl, wobei Alkyl jeweils unabhängig linear, cyclisch oder verzweigt sein können, wobei die genannten Alkyl- und Arylgruppen jeweils unabhängig unsubstituiert oder substituiert sein können, die jeweilige substituierte -(C₆-C₂₀)-Arylgruppe kann mindestens einen oder mehrere Substituenten aufweisen;
wobei die Substituenten an der jeweiligen Arylgruppe unabhängig voneinander ausgewählt sein können aus: -O-(C₁-C₁₂)-Alkyl, -(C₁-C₁₂)-Alkyl-O-(C₁-C₁₂)-Alkyl, -(C₁-C₁₂)-Alkyl, -O-(C₆-C₂₀)-Aryl, -O-(C₁-C₁₂)-Alkyl-O-(C₁-C₁₂)-Alkyl, -O-(C₆-C₂₀)-Aryl-O-(C₁-C₁₂)-Alkyl, -(C₃-C₁₂)-Cycloalkyl, -(C₃-C₁₂)-Heterocycloalkyl, -(C₆-C₂₀)-Aryl, Cyano, -Halogen, -OCOO-(C₁-C₁₂)-Alkyl, -N[(C₁-C₁₂)-Alkyl]₂,
umfassend mindestens den Verfahrensschritt
(i) Umsetzen eines Selenodiaryls der allgemeinen Struktur **XI**, 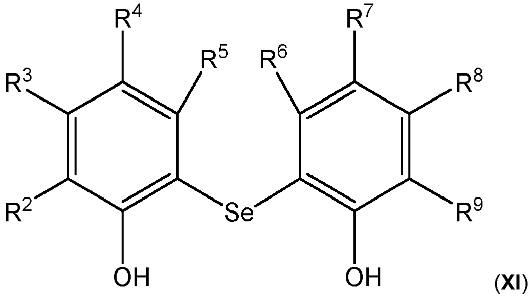 wobei R², R³, R⁴, R⁵, R⁶, R⁷, R⁸ und R⁹ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₂H, -CN, -N[(C₁-C₁₂)-Alkyl]₂, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe, mindestens einen Substituenten aufweisen kann und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, insbesondere wie vorstehend für Struktur **I** definiert,
(ii) mit einer Dihalogenphosphit-Verbindung R¹P(Hal)₂ der Formel **XII**, wobei Hal ausgewählt ist aus Fluor, Chlor, Brom, Jod, bevorzugt sind Chlor und Brom, besonders bevorzugt ist Chlor, wobei R¹ der vorgenannten Definition entsprechen kann, besonders bevorzugt erfolgt die Umsetzung mit einer Verbindung der Formel **XII** ausgewählt aus R¹PCl₂ oder R¹PBr₂,
(iii) und erhalten mindestens eines heterozyklischen Selena-Phosphits der allgemeinen Struktur **I**.

Entsprechend einer bevorzugten Ausführungsvariante des Verfahrens kann ein Selenodiaryl der allgemeinen Struktur **XIa** eingesetzt werden 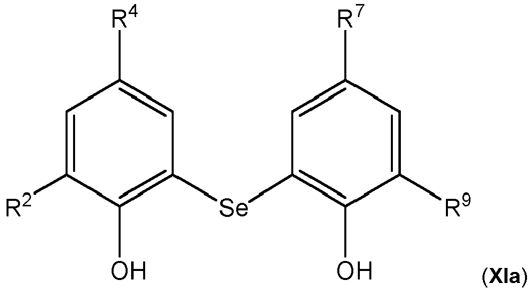 wobei R², R⁴, R⁷ und R⁹ jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen.

Des Weiteren ist es bevorzugt, wenn in dem Verfahren mit dem Selenodiaryl der Struktur **XI** oder **XIa** eine Dihalogenphosphit-Verbindung R¹P(Hal)₂ der Formel **XII** umgesetzt wird, mit Hal ausgewählt aus Fluor, Chlor, Brom, Jod, insbesondere Chlor und Brom, besonders bevorzugt ist Chlor, in der R¹ unabhängig ausgewählt sein kann aus den Strukturen **II**, **III**, **IV**, **V**, **VI**, **VII**, **VIII**, **IX** und **X** wie vorstehend definiert, wobei die Reste R¹⁰, R¹¹, R¹², R¹³, R¹⁴ in der Struktur **II**, R¹⁵, R¹⁶, R¹⁷, R¹⁸, R¹⁹, R²⁰, R²¹ und R²² in Struktur **III**, R²⁴, R²⁵, R²⁶, R²⁷, R²⁸, R²⁹, R³⁰, R³¹, R³² und R³³ in Struktur **IV**, R³⁵, R³⁶, R³⁷, R³⁸, R³⁹ R⁴⁰, R⁴¹, R⁴², R⁴³ und R⁴⁴ in Struktur **V,** R⁴⁶, R⁴⁷, R⁴⁸, R⁴⁹, R⁵⁰, R⁵¹, R⁵², R⁵³, R⁵⁴, R⁵⁵, R⁵⁶, und R⁵⁷ In Struktur **VI**, R⁵⁹, R⁶⁰, R⁶¹, R⁶², R⁶³, R⁶⁴ und R⁶⁵ in Struktur **VII**, sowie R⁶⁶, R⁶⁷, R⁶⁸, R⁶⁹, R⁷⁰, R⁷¹, R⁷², R⁷³ und R⁷⁴ in Struktur **VIII**, in jeder Struktur jeweils unabhängig voneinander ausgewählt sind aus: -H, -(C₁-C₁₂)-Alkyl, -O-(C₁-C₁₂)-Alkyl, -Halogen, und wobei jeweils unabhängig zusätzlich zu den vorgenannten Gruppen in den Strukturen **III**, **IV**, **V** und **VI** R²³, R³⁴, R⁴⁵, R⁵⁸ jeweils unabhängig ausgewählt sind aus -H, -(C₁-C₁₂)-Alkyl, -Halogen und -O-X mit X gleich Schutzgruppe, wobei die Schutzgruppe X ausgewählt ist aus -(C₁-C₁₂)-Alkyl, -(C₁-C₁₂)-Alkyl-O-(C₁-C₁₂)-Alkyl, -(C₆-C₂₀)-Aryl-O-(C₁-C₁₂)-Alkyl, -COO-(C₁-C₁₂)-Alkyl.

Erfindungsgemäss ist die Dihalogenphosphit-Verbindung R¹P(Hal)₂ eine organofunktionelle Dihalogenphosphit-Verbindung.

Die (i) Umsetzung im erfindungsgemässen Verfahren erfolgt vorzugsweise in Gegenwart einer Base, insbesondere eines Amins oder einer Pyridinbase, insbesondere eines Alkylamins, wie Triethylamin oder Dimethylaminobutan, insbesondere Triethylamin.

Ferner erfolgt die (i) Umsetzung vorzugsweise indem das Selenodiaryl der allgemeinen Struktur **XI** mit R¹P(Hal)₂ der Formel **XII** im molaren Verhältnis von 10 : 1 bis 1 : 10 umgesetzt wird, vorzugsweise im Verhältnis von 2 :1 bis 1: 2, besonders bevorzugt 1,5 : 1 bis 1 : 1,5.

Des Weiteren erfolgt die (i) Umsetzung vorzugsweise bei einer Temperatur von -45 °C bis 80 °C, besonders von -15 bis 30°C, insbesondere von -5 bis 5°C.

Gleichfalls Gegenstand der Erfindung ist die Verwendung eines heterozyklischen Selena-Phosphits der Struktur **I**, **Ia** und **Ib** oder der Zusammensetzung umfassen mindestens ein Selena-Phosphit der Struktur **I** erhältlich nach dem Verfahren als Ligand.

Der Begriff Phenol, Aryl, Phosphit wird in dieser Anmeldung als Gattungsbegriff verwendet und umfasst somit auch substituierte Strukturen der genannten Verbindungen.

Ein oder mehrere Substituenten in den vorgenannten Strukturen der Selena-Phosphite und Selenodiarylen umfassen vorzugsweise 1 bis 10 Substituenten, insbesondere 1 bis 3.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl-, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Halogen als Substituent an Alkyl oder Aryl umfasst Fluor, Chlor, Brom und Jod, wobei Chlor und Fluor besonders bevorzugt sind.

Alle Erläuterungen zum Ausdruck -(C₁-C₁₂)-Alkyl in den vorgenannten erfindungsgemäßen Strukturen der Selena-Phosphite und Selenodiarylen gelten auch für die Alkylgruppen in -O-(C₁-C₁₂)-Alkyl, also in -(C₁-C₁₂)-Alkoxy.

Bevorzugt sind unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen in den vorgenannten Strukturen der Selena-Phosphite und Selenodiarylen können in Abhängigkeit von ihrer Kettenlänge, einen oder mehrere Substituenten aufweisen. Die Substituenten sind vorzugsweise unabhängig voneinander ausgewählt aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl. Diese Definition gilt für alle substituierten Alkyl- oder Alkyoxygruppen der vorliegenden Erfindung.

Alle Erläuterungen zum Ausdruck -(C₆-C₂₀)-Aryl in den vorgenannten erfindungsgemäßen Strukturen der Selena-Phosphite und Selenodiarylen gelten auch für die Arylgruppen in -O-(C₆-C₂₀)-Aryl.

Bevorzugt sind unsubstituierte -O-(C₆-C₂₀)-Gruppen.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.
Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 die Ringkohlenstoffatome durch Heteroatome oder heteroatomhaltige Gruppen ersetzt. Die Heteroatome oder die heteroatomhaltigen Gruppen sind vorzugsweise ausgewählt aus -O-, -S-, -N-, -N(=O)-, -C(=O)- oder -S(=O)-. Beispiele für -(C₃-C₁₂)-Heterocycloalkylgruppen sind Tetrahydrothiophenyl, Tetrahydrofuryl, Tetrahydropyranyl und Dioxanyl.

Nachfolgend wird die Erfindung näher an Beispielen erläutert, ohne die Erfindung auf die Ausführungsbeispiele zu beschränken.

### Allgemeine Methoden

### Lösungsmittel und Reagenzien

Alle Reaktionen mit feuchtigkeits- und/oder sauerstoffempfindlichen Substanzen wurden in ausgeheizten Apparaturen unter Argonatmosphäre durchgeführt. Lösungsmittel zur Extraktion und Säulenchromatographie wurden in folgenden Reinheiten verwendet: Dichlormethan (99.9%, Fa. *Walter*, Art.-Nr. BIE 073107033) Ethylacetat (99.5%, Fa. *Walter*, Art.-Nr. BIE 003917025) und *n-*Hexan (95%, Fa. *Walter (Baker),* Art.-Nr. 8669), *n*-Heptan (95%, Fa. *Walter (Baker)*, Art.-Nr. 8662). Andere Lösungsmittel für Extraktion und Säulenchromatographie waren von technischer Qualität und wurden, sofern nicht anders vermerkt, ohne weitere Reinigung eingesetzt. Trockene Lösungsmittel (abs.) wurden mit einem Pure Solv MD-7 System gereinigt und unter Argonatmosphäre aufbewahrt. Benzylbromid wurde vor dem Gebrauch frisch destilliert (17 mbar/82°C). Deuterierte Lösungsmittel wurden von den angegebenen Trockenmitteln destilliert: Dichlormethan-d₂ (Phosphorpentoxid), Toluol-d₈ (1. KOH; 2. Natrium). Für die Synthesen wurden Chemikalien der Firmen *Sigma Aldrich, Alfa Aesar, Acros Organics, Avantor Performance Materials B.V., Merck KGaA* und *ABCR GmbH* & *Co. KG* verwendet. Diese wurden, sofern nicht anders vermerkt, ohne weitere Reinigung eingesetzt.

Filtration: Filtrationen zur Abtrennung von entstandenen Feststoffen wurden mit einer G4-Fritte (Porenweite: 10-16 µm) durchgeführt.

### Analytik

IR-Spektroskopie: Die IR-Spektren wurden mit dem FT-IR-Spektrometer *Nicolet 6700* der Firma *Thermo Electron* aufgenommen. Die Substanzen wurden mittels ATR-Verfahren vermessen.

¹H-NMR-Spektroskopie: Die ¹H-NMR-Spektren wurden mit dem Modell *AV 300* (300 MHz) sowie mit dem Modell *Fourier 300* (300 MHz) der Firma *Bruker* aufgenommen. Die chemischen Verschiebungen sind in Einheiten der δ-Skala angegeben. Die Restprotonensignale der Lösungsmittel (Dichlormethan-d₂: δ = 5.32 ppm, Toluol-d₈: δ = 7.09; 7.00; 6.98; 2.09 ppm) dienten dabei als Standard.

¹³C-NMR-Spektroskopie: Die ¹³C-NMR-Spektren wurden mit den Modellen *AV 300* (75 MHz) und *Fourier 300* (75 MHz) der Firma *Bruker* aufgenommen. Als interner Standard diente das Signal des Lösungsmittels (Dichlormethan-d₂: δ = 54.0 ppm, Toluol-d₈: δ = 137.9; 129.2; 128.3; 125.5; 20.4 ppm) wobei die chemischen Verschiebungen den ¹H-breitbandentkoppelten Spektren entnommen wurden.

⁷⁷Se-NMR-Spektroskopie: Die "Se-NMR-Spektren wurden mit dem *AV 300* (57 MHz) der Firma *Bruker* aufgenommen. Die Spektren wurden ¹H-breitbandentkoppelt vermessen. Die chemischen Verschiebungen sind in ppm angegeben.

Massenspektrometrie: EI-Massespektren wurden am Gerät *Finnigan MAT 95-XP* der Firma *Thermo Electron* sowie ESI-TOF-Massespektren mit dem Modell *6210 Time-of Flight LC*/*MS* der Firma *Agilent* aufgenommen.

### Synthese der Vorstufen:

### Allgemeine Arbeitsvorschrift

8.2 mmol des jeweiligen Phenols werden im entsprechenden Lösungsmittel gelöst (8.2 m). Das Reaktionsgemisch wird erhitzt und 4.9 mmol Selendioxid wird unter Rühren zugegeben. Das Lösungsmittel wird im Vakuum destilliert (Temperatur <70° C). Eine Fritte wird mit 2,5 cm Kieselgel (unten) und 2,5 cm Zeolith (oben) vorbereitet. Der Destillationsrückstand wird im Laufmittel aufgenommen und auf die Filtrationssäule geben. Mit Cyclohexan:Essigester (95:5) wird das Produkt von der Fritte gewaschen und in Fraktionen gesammelt. Die Fraktionen die das Produkt enthalten werden zusammengefasst und destillativ vom Laufmittel befreit. Die erhaltenen Fraktionen werden aus Cyclohexan:Essigester 95:5 umkristallisiert. Dazu wird der feste Rückstand bei 50 °C gelöst und unlösliche Rückstände über eine Glasfritte abfiltriert. Aus der gesättigten Lösung kristallisiert das Reaktionsprodukt bei Raumtemperatur über Nacht. Die erhaltenen Kristalle werden nochmal mit kaltem Cyclohexan gewaschen.

Die Strukturformel zeigt jeweils das bei der Reaktion angefallene Hauptprodukt.

### Bis(3,5-dimethyl-2-hydroxyphenyl)selen; StrukturXI, 1a

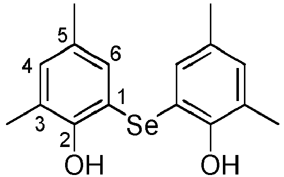

Die Durchführung der Reaktion erfolgt gemäß der allgemeinen Arbeitsvorschrift in einem verschraubbaren Reagenzglas. Hierzu werden 1.00 g (8.2 mmol, 1.0 Äquiv.) 2,4-Dimethylphenol und 0.54 g (4.9 mmol, 0.6 Äquiv.) Selendioxid in 1 mL Pyridin gelöst und erhitzt. Das Produkt wird als farbloser kristalliner Feststoff erhalten.

¹H-NMR (400 MHz, CDCl₃): δ (ppm) = 7.12 (s,2H, 6-H), 6.91 (s, 2H, 4-H), 5.97 (s,2H, OH), 2.23 (s, 6H, 3-CH₃) 2.23 (s, 6H, 5-CH₃); ¹³C-NMR (100 MHz, CDCl₃): δ (ppm) = 151.7 (C-2),133.2 (C-3), 133.1 (C-5), 130.4 (C-4), 124.2 (C-6), 114.9 (C-1), 20.3 (5-CH₃), 16.5 (3-CH₃); ⁷⁷Se-NMR (76 MHz, CDCl₃): δ (ppm) = 163.36 ppm.

### Bis(3-tert-butyl-5-methyl-2-hydroxyphenyl)selen, StrukturXI, 1b

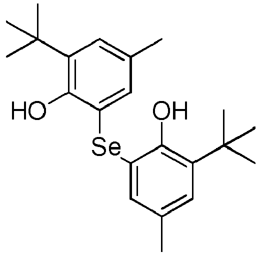

Die Durchführung der Reaktion erfolgt gemäß der allgemeinen Arbeitsvorschrift in einem verschraubbaren Reagenzglas. Dazu wurden 1.32 g (8.0 mmol, 1.0 Äquiv.) 2-*tert*-Butyl-4-methylphenol und 0.54 g (4.9 mmol, 0.6 Äquiv.) Selendioxid in 1 mL Pyridin gelöst und erhitzt.

¹H-NMR (300 MHz, CDCl₃): δ (ppm) = 7.15 (s, 2H, 6-H), 7.05 (s, 2H, 4-H), 5.07 (s,2H, OH), 2.21 (s, 6H, 5-CH₃), 2.21 (s, 18H, 3-C(CH₃)₃; ¹³C-NMR (75 MHz, CDCl₃): δ (ppm) = 152.1, 136.4, 133.4, 120.1, 129.5, 117.2, 35.1, 29.6, 20.8.

### 3,3',5,5'-Tetra-tert-butylbiphenyl-2,2'-diol, Struktur XI, 1c

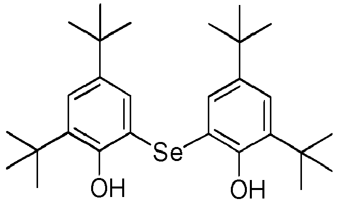

Die Durchführung der Reaktion erfolgt gemäß der allgemeinen Arbeitsvorschrift in einem verschraubbaren Reagenzglas. Dazu wurden 1.67 g (8.2 mmol, 1.0 Äquiv.) 2,4-Di-*tert*-butylphenol und 0.55 g (4.9 mmol, 0.6 Äquiv.) Selendioxid in 1 mL Pyridin gelöst und erhitzt.

¹H-NMR (400 MHz, CDCl₃): δ (ppm) = 7.31 (d, J=2.4 Hz, 2H), 7.29 (d, J=2.4), 6.29 (s, 2H), 1.42 (s, 18H), 1.24 (s, 18H); ¹³C-NMR (75 MHz, CDCl₃): δ (ppm) = 151.7, 143.5, 135.8, 129.8, 125.6, 117.2, 35.4, 34.4, 31.6, 29.7.

### Biphenole

Die Synthese der Biphenole erfolgt analog zu DE102013203865 und DE102013203867.

### Synthese der Chlorophosphite

Die Synthese der Dichlorophosphite, wie Dichlor((-)-menthyloxy)phosphit ist dem Fachmann bekannt und erfolgt nach bekannter Weise. Chlorophosphite lassen sich durch Zusatz von Phosphortrichlorit in Gegenwart einer Base aus den entsprechenden mono-Hydroxyverbindungen herstellen. Für nähere Informationen siehe auch "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.

### Synthese von Dichloro(2,4-di-tert-butylphenoxy)phosphit, Struktur XII, 2a

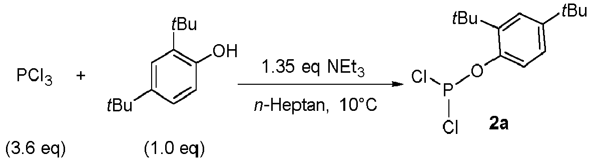

In einem ausgeheizten, unter Argon-Atmosphäre befindlichen 50 mL Schlenkgefäß wurden 629 µL (989 mg, 7.20 mmol, 3.6 eq) Phosphortrichlorid, in 20 mL abs. *n*-Heptan vorgelegt und auf 10°C gekühlt. In einem separaten 10 mL Schlenkgefäß wurden 374 µL (273 mg, 2.70 mmol, 1.35 eq) Triethylamin und 412 mg (2.00 mmol, 1.0 eq)

2,4-Di-*tert*-Butylphenol in 10 mL *n*-Heptan gelöst und tropfenweise über einen Zeitraum von 90 Minuten zum vorgelegten PCl₃ addiert. Es wurde mit 2.0 mL abs. *n*-Heptan nachgespült und 19 h bei RT gerührt. Anschließend wurde das Reaktionsgemisch bis zum vollständigen Abtrennen des entstandenen Niederschlages filtriert und der Feststoff mit 10 mL abs. *n*-Heptan gewaschen. Das Lösungsmittel der hellgelben Lösung wurde unter vermindertem Druck entfernt und das Rohprodukt drei Stunden im Vakuum bei 50°C getrocknet. Es wurden 569 mg (1.86 mmol, 93%, 96%ig) der Titelverbindung **2a** als farbloses Öl erhalten.

IR (ATR): *v̂* (cm⁻¹) = 2958; 2869; 1494; 1398; 1362; 1302; 1210; 1154; 1085; 982; 939; 887; 823; 783; 745; 699; 645; 598; 509; ³¹P-NMR (122 MHz, Toluol-d₈): δ (ppm) = 186.0.

MS (EI): m/z (%) = 306 (10.4) [C₁₄H₂₁Cl₂OP]; 291 (100) [C₁₃H₁₈Cl₂OP]; 271 (2.06) [C₁₄H₂₁ClOP]; HR-MS (EI): ber. für C₁₄H₂₁ClOP: 306.07016, gef.: 306.06994;

ber. für C₁₄H₂₁³⁷ClOP: 308.06721, gef.: 308.06731; C₁₄H₂₁Cl₂OP (306.07 g/mol).

### Umsetzung von tert-Butyl-(3,3'-di-tert-butyl-2'-hydroxy-5,5'-dimethoxy-[1,1'-biphenyl]-2-yl)-carbonat mit Phosphortrichlorid:

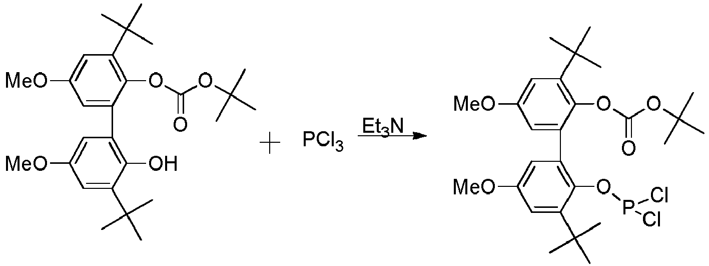

In einem sekurierten 250 ml Schlenkkolben wurden 12 g (0,026 mol) tert-Butyl-(3,3'-di-tert-butyl-2'-hydroxy-5,5'-dimethoxy-[1,1'-biphenyl]-2-yl)-carbonat unter Rühren in 120 ml getrocknetem Toluol und 12,8 ml (0,091 mol) Triethylamin gelöst.

In einem zweiten 500 ml Schlenkkolben wurden zunächst 100 ml getrocknetes Toluol mit 8,1 ml (0,091 mol) Phosphortrichlorid verrührt. Anschließend wurde die Phosphortrichlorid-Toluol-Lösung zu der zuvor hergestellten Carbonat-Amin-Toluol-Lösung innerhalb von 30 Minuten bei Raumtemperatur zugetropft. Nach vollständiger Zugabe wurde für 30 Minuten auf 80 °C erwärmt und über Nacht auf Raumtemperatur abgekühlt. Am nächsten Morgen wurde die Mischung filtriert, mit 50 ml getrocknetem Toluol nachgewaschen und das Filtrat bis zur Trockne eingeengt. Das Zielprodukt konnte als Feststoff (13,1 g, 89%) erhalten werden. ³¹P-NMR (202,4 MHz, Toluol-d₈): 203,2 und 203,3 ppm.

### Darstellung von Biphenyl-3,3',5,5'-Tetratert.-butyl-2-hydroxy-2'-dichlorophosphit

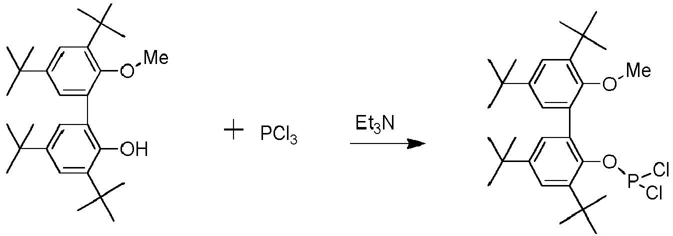

In einem sekurierten 250 ml Schlenkkolben wurde 10,62 g (0,025 mol) 3,3',5,5'-Tetratert.-butyl-2-hydroxy-2'-methoxy-biphenyl unter Rühren in 50 ml getrocknetem Toluol gelöst und mit 3,5 ml (0,025 mol) Triethylamin versetzt. Zu der erhaltenen Lösung tropft man bei Raumtemperatur unter kräftigem Rühren 2,2 ml (0,025 mol) Phosphortrichlorid hinzu und erhitzt dann für 4 Stunden auf 105 °C. Zur Aufarbeitung filtriert man das ausgefallene Ammoniumchlorid ab und wäscht 2 mal mit 25 ml Toluol nach. Das Filtrat wird bis zur Trockne eingeengt. Das Produkt konnte in 63%iger Ausbeute erhalten werden.

Analog zur Herstellung des Dichloro(2,4-di-*tert*-butylphenoxy)phosphit können entsprechend Phenole, 1-Naphthole, 2-Naphthole, Antracen-Derivate, wie 9-Hydroxyantracen, und Cycloalkanol-Verbindungen hergestellt werden.

### Die Synthese der Selen-Phosphite

### Synthese von 6-(2,4-Di-tert-butylphenoxy)-2,4,8,10-tetramethyldibenzo[d,g][1,3,6,2]dioxaselenaphosphocin, Struktur I; 3a

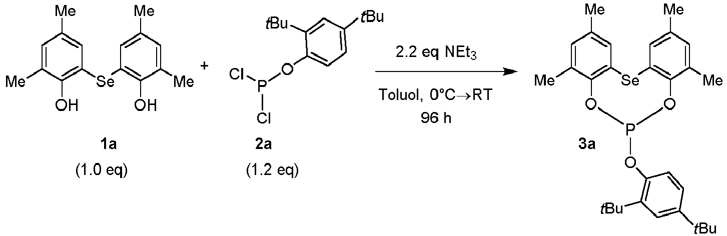

In einem ausgeheizten, unter Argonatmosphäre befindlichen 50 mL Schlenkgefäß wurden 191 mg (0.624 mmol, 1.2 eq, 96%ig) Dichloro(2,4-di-*tert*-butylphenoxy)phosphit **2a** in 5.0 mL abs. Toluol vorgelegt und auf 0°C gekühlt. In einem separaten 10 mL Schlenkgefäß wurden 168 mg (0.522 mmol, 1.0 eq) Selenodiphenol **1a** und 159 µL (116 mg, 1.14 mmol, 2.2 eq) Triethylamin in 2.0 mL abs. Toluol gelöst. Die resultierende hellgelbe Lösung wurde anschließend zum vorgelegten Dichlorophosphit **2a** tropfenweise addiert, wobei die Bildung eines farblosen Niederschlages verzeichnet wurde. Es wurde mit 2.0 mL abs. Toluol nachgespült und für 48 h bei RT gerührt. Die Reaktionsmischung wurde zur vollständigen Abtrennung des entstandenen Niederschlages filtriert, der Feststoff mit 10 mL abs. Toluol gewaschen und das Lösungsmittel unter vermindertem Druck entfernt. Nach Kristallisation in 10 mL abs. *n*-Heptan wurden 191 mg (0.343 mmol, 66%, 99.9% im ³¹P-NMR) der Titelverbindung **3a** als farbloser Feststoff erhalten.

IR(ATR): *v̂* (cm⁻¹) = 3425; 2956; 2917; 2865; 1604; 1492; 1464; 1399; 1377; 1360; 1273; 1248; 1208; 1192; 1119; 1084; 1013; 958; 914; 887; 848; 810; 772; 729; 703; 680; 669; 645; 581; 527; 512; 497; 412; ¹H-NMR (300 MHz, Toluol-d₈): δ (ppm) = 7.68-7.46 (m, 2H Ar-CH); 7.40-7.31 (m, 2H Ar-CH); 7.05 (dd, *J* = 8.4 Hz, *J* = 2.5 Hz, 1H Ar-CH); 6.64 (ddd, *J* = 2.3 Hz, *J* = 1.3 Hz, *J* = 0.7 Hz, 2H, Ar-CH); 2.13 (s, 6H, -CH₃); 1.97-1.95 (m, 6H, -CH₃); 1.65 (s, 9H, -C(CH₃)₃); 1.31 (s, 9H,-C(CH₃)₃); ¹³C-NMR (75 MHz, Toluol-d₈):
δ (ppm) = 152.9 (d, *J* = 5.4 Hz); 150.1 (d, *J* = 4.0 Hz); 145.8; 139.6 (d, *J* = 2.5 Hz); 134.3; 133.8; 133.1; 130.10; 124.4; 124.0; 120.3 (d, *J* = 17.1 Hz); 120.0 (d, *J* = 4.2 Hz); 35.41; 34.57; 31.63; 30.42; 20.16; 17.35; ³¹P-NMR (122 MHz, Toluol-d₈): δ (ppm) = 132.6 (*J*_{P-Se} = 62.3 Hz); ⁷⁷Se-NMR (57 MHz, Toluol-d₈): δ (ppm) = 323.0 (*J*_{Se-P} = 62.3 Hz); C₃₀H₃₇O₃PSe (556.16 g/mol).

## Patentansprüche

1. Verbindung eines heterozyklischen Selena-Phosphites, welches eine allgemeine Struktur (I) aufweist 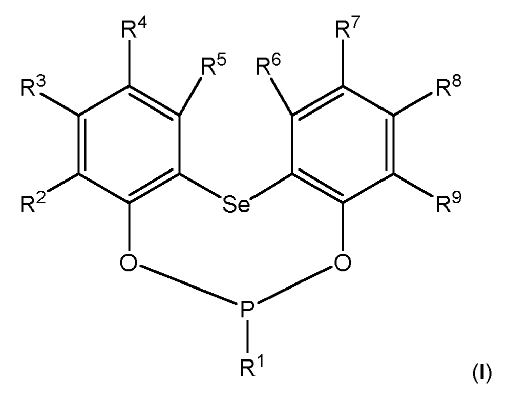 wobei R², R³, R⁴, R⁵, R⁶, R⁷, R⁸ und R⁹ jeweils unabhängig voneinander ausgewählt sind aus: - H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkylgruppen jeweils unabhängig linear, verzweigt oder cyclisch sind, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe, mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei -R¹ unabhängig ausgewählt ist aus -O-(C₆-C₂₀)-Aryl, -O-(C₆-C₂₀)-Aryl-(C₁-C₁₂)-Alkyl, -O-(C₆-C₂₀)-Aryl-O-(C₁-C₁₂)-Alkyl, -O-(C₆-C₂₀)-Aryl-(C₆-C₂₀)-Aryl, -O-(C₆-C₂₀)-Aryl-O-(C₆-C₂₀)-Aryl, -O-(C₁-C₁₂)-Alkyl, -O-(C₁-Cl₂)-Alkyl-O-(C₁-C₁₂)-Alkyl, -O-(C₁-C₁₂)-Alkyl-(C₆-C₂₀)-Aryl, -O-(C₃-C₁₂)-Cycloalkyl wobei die Alkylgruppen linear, verzweigt oder cyclisch sind, wobei die genannten Alkyl- und Arylgruppen jeweils unabhängig unsubstituiert oder substituiert sind, die jeweilige substituierte - (C₆-C₂₀)-Arylgruppe weist mindestens einen oder mehrere Substituenten auf,
wobei die Substituenten an der jeweiligen Arylgruppe unabhängig voneinander ausgewählt sind aus: -O-(C₁-C₁₂)-Alkyl, -(C₁-C₁₂)-Alkyl-O-(C₁-C₁₂)-Alkyl, -(C₁-C₁₂)-Alkyl, -O-(C₆-C₂₀)-Aryl, -O-(C₁-Cl₂)-Alkyl-O-(C₁-C₁₂)-Alkyl, -O-(C₆-C₂₀)-Aryl-O-(C₁-C₁₂)-Alkyl, -(C₃-C₁₂)-Cycloalkyl, -(C₃-C₁₂)-Heterocycloalkyl, -(C₆-C₂₀)-Aryl, Cyano, -Halogen, -OCOO-(C₁-C₁₂)-Alkyl, -N[(C₁-C₁₂)-Alkyl]₂.

2. Verbindung nach Anspruch 1, **dadurch gekennzeichnet, dass** im heterozyklischen Selena-Phosphit der allgemeinen Struktur (**I**) R¹ ausgewählt ist aus den Strukturen (**II**), (**III**), (**IV**), (**V**), (**VI**), (**VII**), (**VIII**), (**IX**) und (**X**) 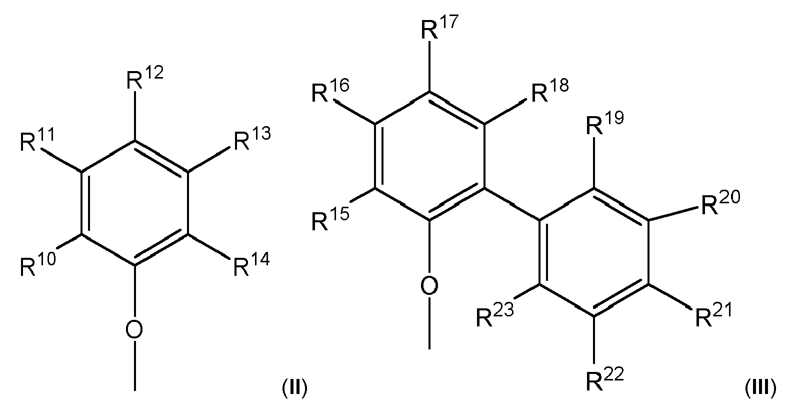 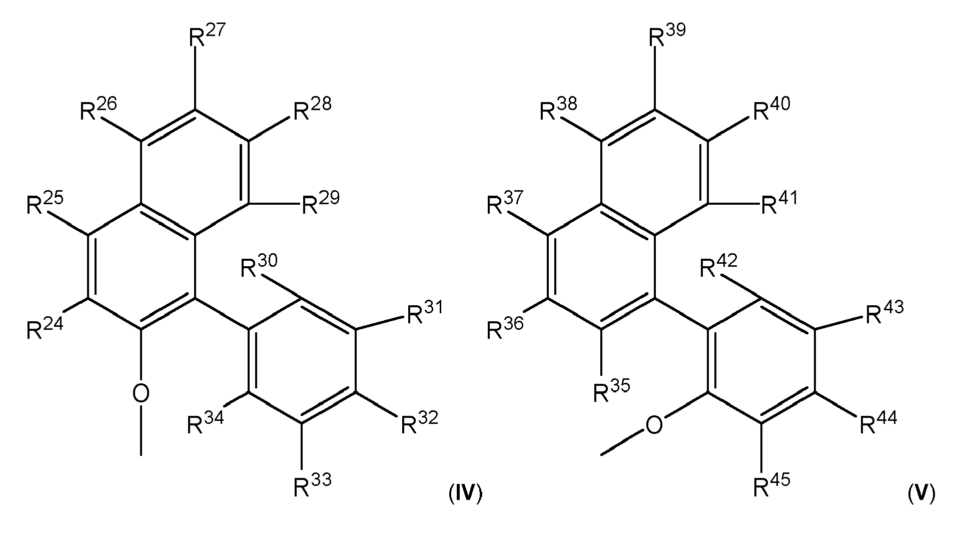 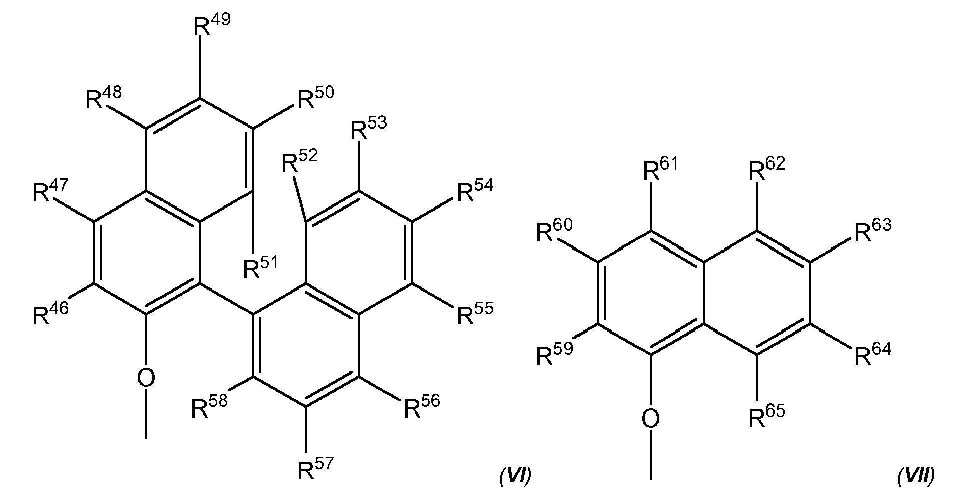 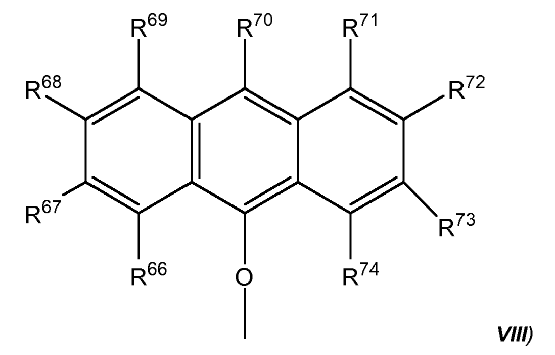 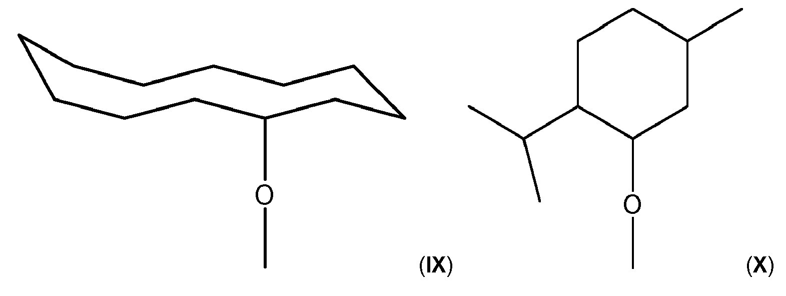 wobei die Reste
R¹⁰, R¹¹, R¹², R¹³, R¹⁴ in der Struktur (**II**),
R¹⁵ R¹⁶, R¹⁷, R¹⁸, R¹⁹ R²⁰, R²¹, R²² und R²³ in Struktur (**III**),
R²⁴, R²⁵, R²⁶, R²⁷, R²⁸, R²⁹, R³⁰, R³¹, R³², R³³ und R³⁴ in Struktur (**IV**),
R³⁵, R³⁶, R³⁷, R³⁸, R³⁹, R⁴⁰, R⁴¹, R⁴², R⁴³, R⁴⁴ und R⁴⁵ in Struktur (**V**),
R⁴⁶, R⁴⁷, R⁴⁸, R⁴⁹, R⁵⁰, R⁵¹, R⁵², R⁵³, R⁵⁴, R⁵⁵, R⁵⁶, R⁵⁷ und R⁵⁸ in Struktur (**VI**),
R⁵⁹, R⁶⁰, R⁶¹, R⁶², R⁶³, R⁶⁴ und R⁶⁵ in Struktur (**VII**), sowie
R⁶⁶, R⁶⁷, R⁶⁸, R⁶⁹, R⁷⁰, R⁷¹, R⁷², R⁷³ und R⁷⁴ in Struktur (**VIII**),
in jeder Struktur jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkyl- und Arylgruppen jeweils unabhängig unsubstituiert oder substituiert sind, wobei die jeweilige substituierte -(C₁-C₁₂)-Alkylgruppe und die jeweilige substituierte -(C₆-C₂₀)-Arylgruppe mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, wobei in den Strukturen (**III**), (**IV**), (**V**) und (**VI**) R²³, R³⁴, R⁴⁵, R⁵⁸ jeweils unabhängig zusätzlich ausgewählt sind aus -O-X mit X gleich Schutzgruppe, wobei die Schutzgruppe X ausgewählt ist aus -(C₁-C₁₂)-Alkyl, -(C₁-C₁₂)-Alkyl-O-(C₁-C₁₂)-Alkyl, -(C₆-C₂₀)-Aryl-O-(C₁-C₁₂)-Alkyl, -COO-(C₁-C₁₂)-Alkyl.

3. Verbindung nach Anspruch 1, **dadurch gekennzeichnet, dass** das heterozyklische Selena-Phosphit die allgemeine Struktur (**Ia**) aufweist 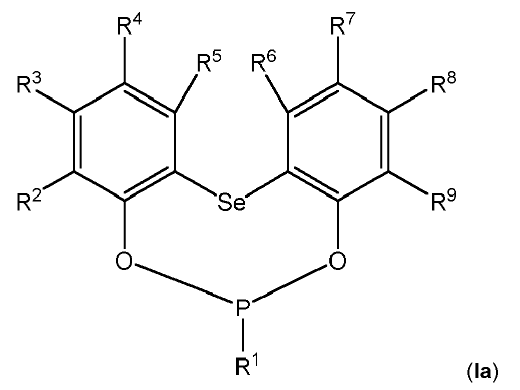 wobei R², R³, R⁴, R⁵, R⁶, R⁷, R⁸ und R⁹ jeweils unabhängig voneinander ausgewählt sind aus: - H, -(C₁-C₁₂)-Alkyl, -O-(C₁-C₁₂)-Alkyl, -(C₆-C₂₀)-Aryl, -O-(C₆-C₂₀)-Aryl, -Halogen, und
-R¹ im heterozyklischen Selena-Phosphit der allgemeinen Struktur (**Ia**) unabhängig ausgewählt ist aus den Strukturen (**II**), (**III**), (**IV**), (**V**), (**VI**), (**VII**), (**VIII**), (**IX**) und (**X**) 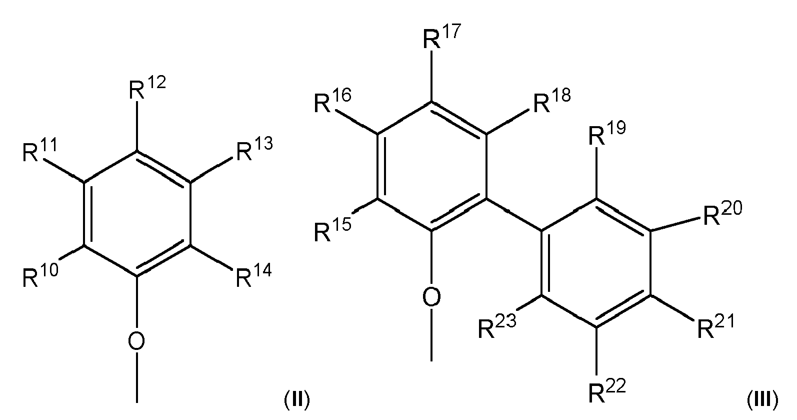 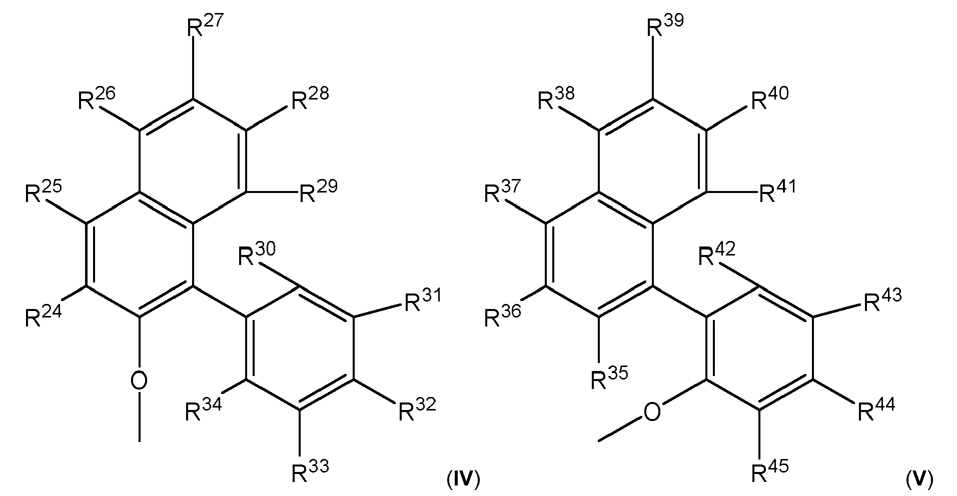 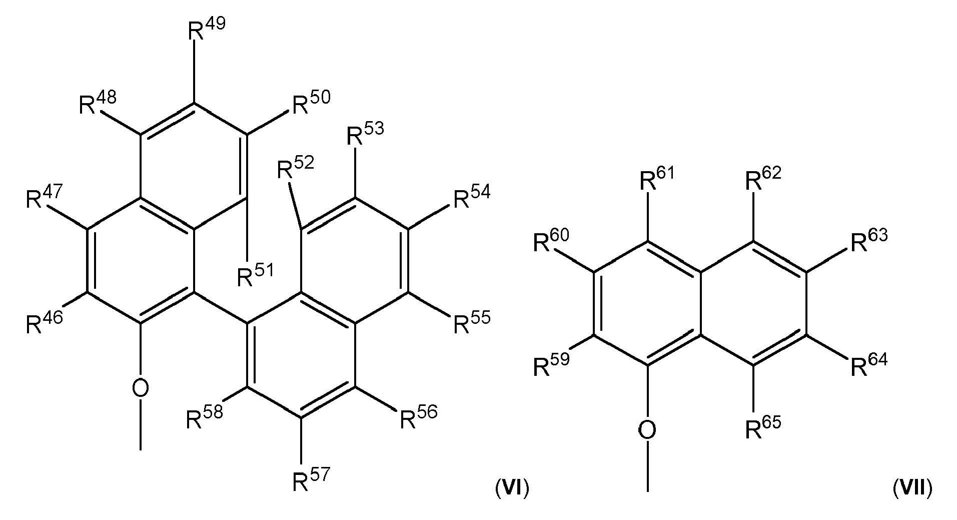 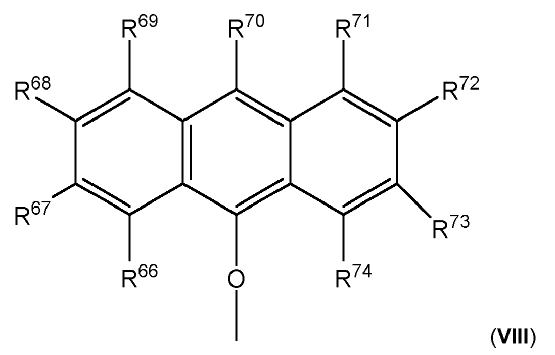 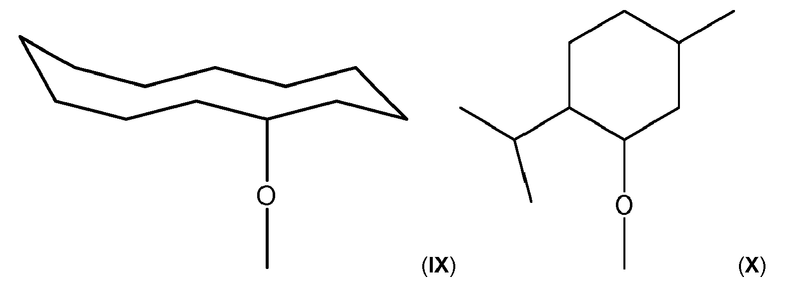 wobei die Reste
R¹⁰, R¹¹, R¹², R¹³, R¹⁴ in der Struktur (**II**),
R¹⁵, R¹⁶, R¹⁷ R¹⁸, R¹⁹, R²⁰, R²¹ und R²² in Struktur (**III**),
R²⁴, R²⁵, R²⁶, R²⁷, R²⁸, R²⁹, R³⁰, R³¹, R³² und R³³ in Struktur (**IV**),
R³⁵, R³⁶, R³⁷, R³⁸, R³⁹, R⁴⁰, R⁴¹, R⁴², R⁴³ und R⁴⁴ in Struktur (**V**),
R⁴⁶, R⁴⁷, R⁴⁸, R⁴⁹, R⁵⁰, R⁵¹, R⁵², R⁵³, R⁵⁴, R⁵⁵, R⁵⁶ und R⁵⁷ in Struktur (**VI**), R⁵⁹, R⁶⁰, R⁶¹, R⁶², R⁶³, R⁶⁴ und R⁶⁵ in Struktur (**VII**), sowie
R⁶⁶, R⁶⁷, R⁶⁸, R⁶⁹, R⁷⁰, R⁷¹, R⁷², R⁷³ und R⁷⁴ in Struktur (**VIII**),
in jeder Struktur jeweils unabhängig voneinander ausgewählt sind aus: -H, -(C₁-C₁₂)-Alkyl, -O-(C₁-C₁₂)-Alkyl, -Halogen, und
wobei jeweils unabhängig zusätzlich zu den vorgenannten Gruppen in den Strukturen (**III**), (**IV**), (**V**) und (**VI**) R²³, R³⁴, R⁴⁵, R⁵⁸ jeweils unabhängig ausgewählt sind aus -H, -(C₁-C₁₂)-Alkyl, -Halogen und -O-X mit X gleich Schutzgruppe, wobei die Schutzgruppe X ausgewählt ist aus -(C₁-C₁₂)-Alkyl, -(C₁-C₁₂)-Alkyl-O-(C₁-C₁₂)-Alkyl, -(C₆-C₂₀)-Aryl-O-(C₁-C₁₂)-Alkyl, -O-COO-(C₁-C₁₂)-Alkyl.

4. Verbindung nach Anspruch 3, **dadurch gekennzeichnet, dass**
das heterozyklische Selena-Phosphit der allgemeinen Struktur (**Ia**) ausgewählt ist aus mindestens einer Verbindung der Struktur (**Ib**) mit R¹ entsprechend der Definition nach Anspruch 3 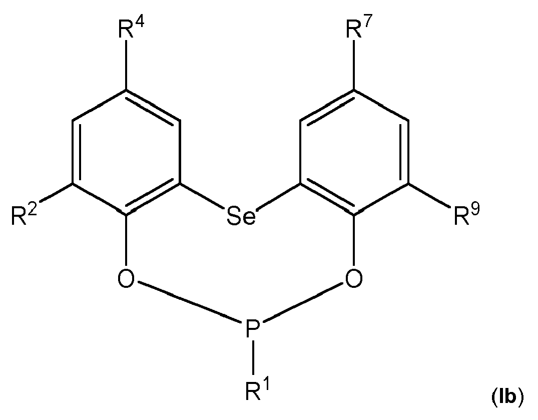 wobei R², R⁴, R⁷ und R⁹ jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkylgruppen jeweils unabhängig linear, verzweigt oder cyclisch sind.

5. Verbindung nach einem der Ansprüche 1 bis 4, **dadurch gekennzeichnet, dass** im heterozyklischen Selena-Phosphit der allgemeinen Struktur (**I**), R², R³, R⁴, R⁵, R⁶, R⁷, R⁸ und R⁹ jeweils unabhängig voneinander ausgewählt sind aus: -H, -(C₁-C₁₂)-Alkyl, -O-(C₁-C₁₂)-Alkyl, und -R¹ im heterozyklischen Selena-Phosphit der allgemeinen Struktur (**I**) unabhängig ausgewählt ist aus den Strukturen (**II**), (**III**), (**IV**), (**V**), (**VI**), (**VII**), (**VIII**), (**IX**) und (**X**) wobei die Reste R¹⁰, R¹¹, R¹², R¹³, R¹⁴ in der Struktur (**II**), R¹⁵, R¹⁶, R¹⁷, R¹⁸, R¹⁹, R²⁰, R²¹ und R²² in Struktur (**III**), R²⁴, R²⁵, R²⁶, R²⁷, R²⁸, R²⁹, R³⁰, R³¹, R³² und R³³ in Struktur (**IV**), R³⁵, R³⁶, R³⁷, R³⁸, R³⁹, R⁴⁰, R⁴¹, R⁴², R⁴³ und R⁴⁴ in Struktur (**V**), R⁴⁶, R⁴⁷, R⁴⁸, R⁴⁹, R⁵⁰, R⁵¹, R⁵², R⁵³, R⁵⁴, R⁵⁵, R⁵⁶, und R⁵⁷ in Struktur (**VI**), R⁵⁹, R⁶⁰, R⁶¹, R⁶², R⁶³, R⁶⁴ und R⁶⁵ In Struktur (**VII**), sowie R⁶⁶, R⁶⁷, R⁶⁸, R⁶⁹, R⁷⁰, R⁷¹, R⁷², R⁷³ und R⁷⁴ in Struktur (**VIII**), in jeder Struktur jeweils unabhängig voneinander ausgewählt sind aus: -H, -(C₁-C₁₂)-Alkyl, -O-(C₁-C₁₂)-Alkyl, und wobei jeweils unabhängig zusätzlich zu den vorgenannten Gruppen in den Strukturen (**III**), (**IV**), (**V**) und (**VI**) R²³, R³⁴, R⁴⁵, R⁵⁸ jeweils unabhängig ausgewählt sind aus -H und -O-X mit X gleich Schutzgruppe, wobei die Schutzgruppe X ausgewählt ist aus -(C₁-C₁₂)-Alkyl, -(C₁-C₁₂)-Alkyl-O-(C₁-C₁₂)-Alkyl, -(C₆-C₂₀)-Aryl-O-(C₁-C₁₂)-Alkyl, -COO-(C₁-C₁₂)-Alkyl.

6. Verfahren zur Herstellung mindestens eines heterozyklischen Selena-Phosphites der allgemeinen Struktur (**I**) 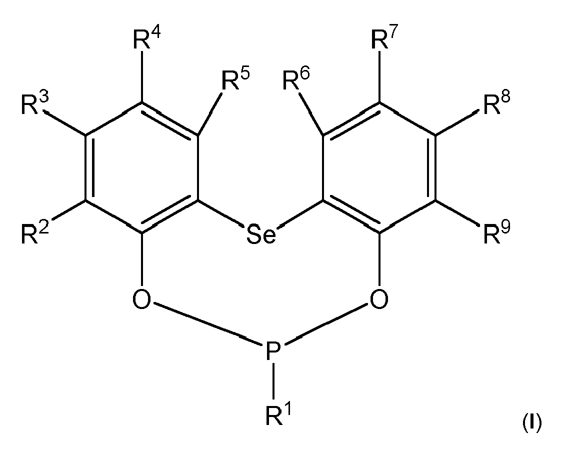 wobei R², R³, R⁴, R⁵, R⁶, R⁷, R⁸ und R⁹ jeweils unabhängig voneinander ausgewählt sind aus: - H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkylgruppen jeweils unabhängig linear, verzweigt oder cyclisch sind, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe, mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei -R¹ unabhängig ausgewählt ist aus -O-(C₆-C₂₀)-Aryl, -O-(C₆-C₂₀)-Aryl-(C₁-C₁₂)-Alkyl, -O-(C₆-C₂₀)-Aryl-O-(C₁-C₁₂)-Alkyl, -O-(C₆-C₂₀)-Aryl-(C₆-C₂₀)-Aryl, -O-(C₆-C₂₀)-Aryl-O-(C₆-C₂₀)-Aryl, -O-(C₁-C₁₂)-Alkyl, -O-(C₁-Cl₂)-Alkyl-O-(C₁-C₁₂)-Alkyl, -O-(C₁-C₁₂)-Alkyl-(C₆-C₂₀)-Aryl, -O-(C₃-C₁₂)-Cycloalkyl, wobei Alkyl jeweils unabhängig linear, cyclisch oder verzweigt ist, wobei die genannten Alkyl- und Arylgruppen jeweils unabhängig unsubstituiert oder substituiert sind, die jeweilige substituierte -(C₆-C₂₀)-Arylgruppe weist mindestens einen oder mehrere Substituenten auf;
wobei die Substituenten an der jeweiligen Arylgruppe unabhängig voneinander ausgewählt sind aus: -O-(C₁-C₁₂)-Alkyl, -(C₁-C₁₂)-Alkyl-O-(C₁-C₁₂)-Alkyl, -(C₁-C₁₂)-Alkyl, -O-(C₆-C₂₀)-Aryl, -O-(C₁-C₁₂)-Alkyl-O-(C₁-C₁₂)-Alkyl, -O-(C₆-C₂₀)-Aryl-O-(C₁-C₁₂)-Alkyl, -(C₃-C₁₂)-Cycloalkyl, -(C₃-C₁₂)-Heterocycloalkyl, -(C₆-C₂₀)-Aryl, Cyano, -Halogen, -O-COO-(C₁-C₁₂)-Alkyl, -N[(C₁-C₁₂)-Alkyl]₂,
umfassend mindestens den Verfahrensschritt
(i) Umsetzen eines Selenodiaryls der allgemeinen Struktur (**XI**) 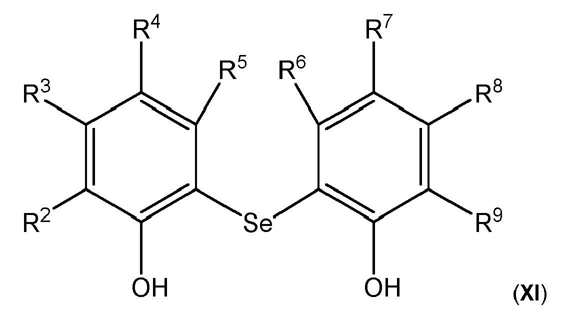 wobei R², R³, R⁴, R⁵, R⁶, R⁷, R⁸ und R⁹ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkylgruppen jeweils unabhängig linear, verzweigt oder cyclisch sind, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe, mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
(ii) mit einer Dihalogenphosphit-Verbindung R¹P(Hal)₂ der Formel (**XII**), wobei Hal ausgewählt ist aus Fluor, Chlor, Brom, Jod, wobei R¹ der vorgenannten Definition entspricht,
(iii) und erhalten mindestens eines heterozyklischen Selena-Phosphits der allgemeinen Struktur (**I**).

7. Verfahren nach Anspruch 6, **dadurch gekennzeichnet, dass**
das Selenodiaryl der allgemeinen Struktur (**XI**) einer Verbindung der Struktur (**XIa**) entspricht 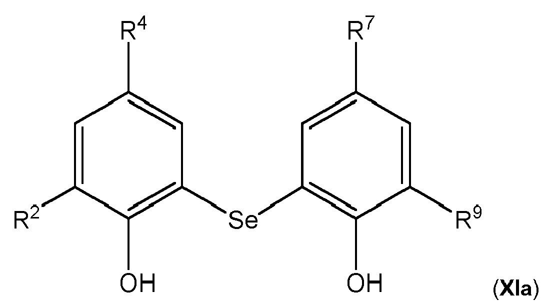 wobei R², R⁴, R⁷ und R⁹ jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkylgruppen jeweils unabhängig linear, verzweigt oder cyclisch sind.

8. Verfahren nach Anspruch 6 oder 7, **dadurch gekennzeichnet, dass** in der Dihalogenphosphit-Verbindung R¹P(Hal)₂ der Formel (**XII**) Hal ausgewählt ist aus Fluor, Chlor, Brom, Jod, und R¹ unabhängig ausgewählt ist aus den Strukturen (**II**), (**III**), (**IV**), (**V**), (**VI**), (**VII**), (**VIII**), (**IX**) **und** (**X**) 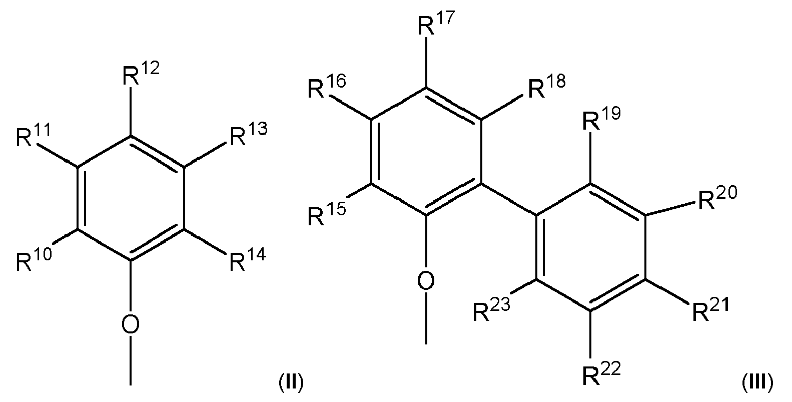 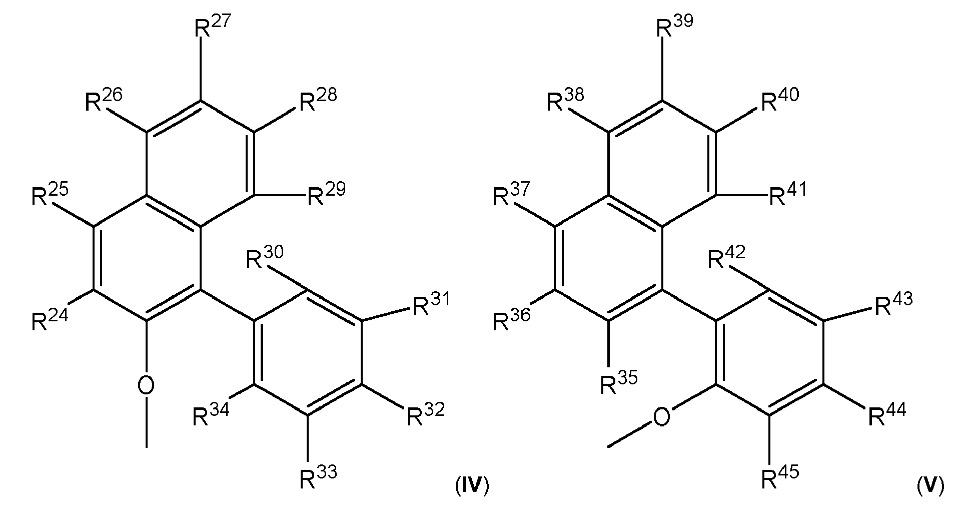 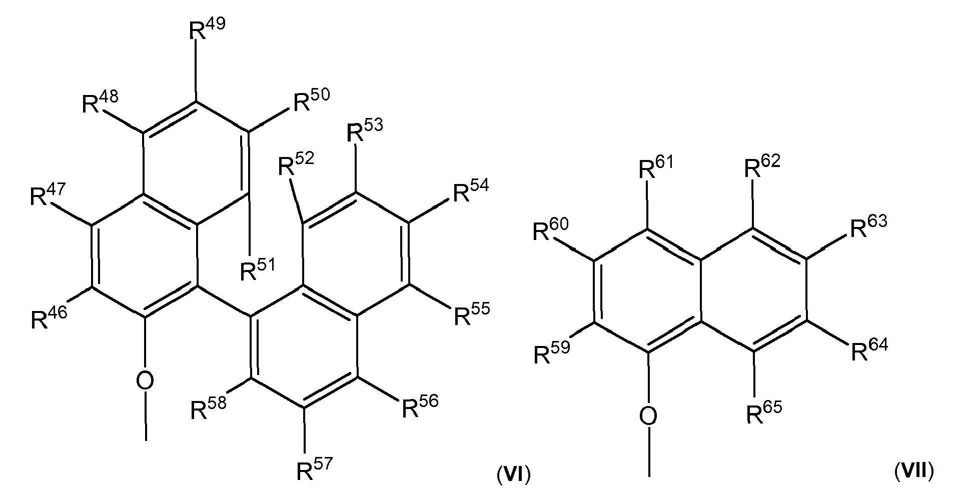 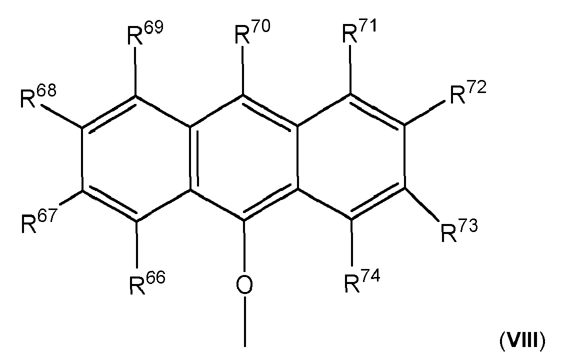 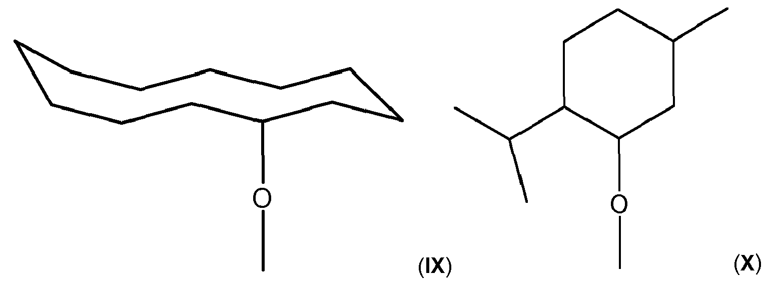 wobei die Reste
R¹⁰, R¹¹, R¹², R¹³, R¹⁴ in der Struktur (**II**),
R¹⁵, R¹⁶, R¹⁷, R¹⁸, R¹⁹ R²⁰, R²¹ und R²² in Struktur (**III**),
R²⁴, R²⁵, R²⁶, R²⁷, R²⁸, R²⁹, R³⁰, R³¹, R³² und R³³ in Struktur (**IV**),
R³⁵, R³⁶, R³⁷, R³⁸, R³⁹, R⁴⁰, R⁴¹, R⁴², R⁴³ und R⁴⁴ in Struktur (**V**),
R⁴⁶, R⁴⁷, R⁴⁸, R⁴⁹, R⁵⁰, R⁵¹, R⁵², R⁵³, R⁵⁴, R⁵⁵, R⁵⁶ und R¹⁷ in Struktur (**VI**),
R⁵⁹, R⁶⁰, R⁶¹, R⁶², R⁶³, R⁶⁴ und R⁶⁵ in Struktur (**VII**), sowie
R⁶⁶, R⁶⁷, R⁶⁸, R⁶⁹, R⁷⁰, R⁷¹, R⁷², R⁷³ und R⁷⁴ in Struktur (**VIII**),
jeweils unabhängig voneinander ausgewählt sind aus: -H, -(C₁-C₁₂)-Alkyl, -O-(C₁-C₁₂)-Alkyl, -Halogen, und
wobei jeweils unabhängig zusätzlich zu den vorgenannten Gruppen in den Strukturen (**III**), (**IV**), (**V**) und (**VI**) R²³, R³⁴, R⁴⁵, R⁵⁸ jeweils unabhängig ausgewählt sind aus -H, -(C₁-C₁₂)-Alkyl, -Halogen und -O-X mit X gleich Schutzgruppe, wobei die Schutzgruppe X ausgewählt ist aus -(C₁-C₁₂)-Alkyl, -(C₁-C₁₂)-Alkyl-O-(C₁-C₁₂)-Alkyl, -(C₆-C₂₀)-Aryl-O-(C₁-C₁₂)-Alkyl, -COO-(C₁-C₁₂)-Alkyl.

9. Verfahren nach einem der Ansprüche 6 bis 8,
wobei (i) die Umsetzung in Gegenwart einer Base erfolgt, insbesondere eines Amins oder Pyridinbase, bevorzugt eines Alkylamins.

10. Verfahren nach einem der Ansprüche 6 bis 9, **dadurch gekennzeichnet,**
**dass** das Selenodiaryl der allgemeinen Struktur (**XI**) mit R¹P(Hal)₂ der Formel (**XII**) im molaren Verhältnis von 10 : 1 bis 1 : 10 umgesetzt wird, vorzugsweise im Verhältnis von 2 :1 bis 1: 2.

11. Verfahren nach einem der Ansprüche 6 bis 10, **dadurch gekennzeichnet,**
**dass** R¹P(Hal)₂ der Formel (**XII**) R¹PCl₂ oder R¹PBr₂ ist.

12. Verfahren nach einem der Ansprüche 6 bis 11,
wobei (i) die Umsetzung im Temperaturbereich von -45 bis 80 °C erfolgt, insbesondere von - 15 bis 30°C.

13. Verwendung eines heterozyklischen Selena-Phosphits nach einem der Ansprüche 1 bis 5 oder erhältlich nach einem der Ansprüche 6 bis 12 als Ligand.
